# EUROPEAN PATENT APPLICATION

(11) **EP 4 295 901 A2**
(43) Date of publication of application: **27.12.2023**
(21) Application number: 23195910.7
(22) Date of filing: 17.06.2019
(51) Int. Cl.: A61P 1/00

(54) **FISH PROTEIN HYDROLYSATE POWDER AND A COMPOSITION COMPRISING SAID POWDER FOR USE AS A MEDICAMENT**

(30) Priority: 20.06.2018 US 201862687665 P
(62) Divisional of application: 19737924.1
(71) Applicant: Hofseth Biocare ASA, 6005 Ålesund (NO)
(72) Inventor: FRAMROZE, Bomi Patel, Menlo Park, CA 94025 (US)
(74) Representative: Zacco Norway AS

(57) **Abstract**

An enzymatic hydrolysis process for producing a fish protein hydrolysate powder is provided. Further provided is use of the fish protein hydrolysate powder obtained and a composition comprising said powder as a medicament, preferably for the prophylaxis or treatment of oxidative damage of the gastro-intestinal and central nervous system.

## Description

### Field Of The Invention :

This invention relates to an enzymatic hydrolysis process for producing a fish protein hydrolysate powder. Further the invention relates to the use of the fish protein hydrolysate powder obtained by the process and a composition comprising said powder as a medicament, preferably for the prophylaxis or treatment of oxidative damage of the gastro-intestinal and central nervous system.

This invention relates to a fish protein hydrolysate and its constituent peptides intended for regulating the function of human oxidation protective genes which provide protection against oxidative damage in various organs and tissues. More specifically, this invention relates to one or more peptide compounds with oxidation protective gene regulating activity incorporated into an oral formulation. This invention also relates to a method for protecting the gastro-intestinal and central nervous system from oxidative damage, which comprises orally ingesting an effective amount of the fish protein hydrolysate or one or more of its constituent peptides.

### Background Of The Invention:

The up/down regulation of one or more oxidative stress-related genes has been proposed as a possible mechanism that can confer cytoprotection to tissues exposed to oxidative injury. While molecular oxygen is essential for the survival of almost all eukaryotes, its processing under physiological conditions generates reactive oxygen species (ROS) such as hydrogen peroxide, superoxide, peroxynitrite, and hydroxyl radicals, as metabolic by-products. In the absence of an adequate defense mechanism, the accumulation of ROS and electrophiles leads to cell membrane and DNA damage, mutagenicity, degeneration of tissues, premature aging, apoptotic cell death and cancers. [Ward, J. F. (1994) The complexity of DNA damage: relevance to biological consequences. Int. J. Radiat Biol. 66, 427-432 ; Goetz, M. E., and Luch, A. (2008) Reactive species: A cell damaging rout assisting to chemical carcinogens. Cancer Lett. 266, 73-83 ; Strassburg, C. P., Manns, M. P., and Tukey, R. H. (1997) Differential Down-Regulation of the UDP-Glucuronosyltransferase 1A Locus Is an Early Event in Human Liver and Biliary Cancer. Cancer Res. 57, 2979-2985]
To combat this oxidative stress, mammalian cells have developed an array of inducible defensive gene activations which leads to a neutralization of the oxidative stress events, reduced ROS and hence increased cell survival. [Dhakshinamoorthy, S., Long, D. J., Jaiswal, A. K. (2000) Antioxidant Regulation of Genes Encoding Enzymes That Detoxify Xenobiotics and Carcinogens. Curr. Top. Cell Regul. 36, 201-216 ; Jaiswal, A. K. (2000) Regulation of genes encoding NAD(P)H: quinone oxidoreductases. Free Radic Biol. Med. 29, 254-262]
Enzymes with antioxidant properties, capable of inactivating ROS and preventing ROS-initiated reactions include superoxide dismutases, catalase, and glutathione peroxidase. They belong to the group referred to as "direct" Phase 1 antioxidant enzymes. [Auten, R. L., O'Reilly, M. A., Oury, T. D., Nozik-Grayck, E., and Whorton, M. H. (2006) Transgenic extracellular superoxide dismutase protects postnatal alveolar epithelial proliferation and development during hyperoxia. Am. J. Physiol. Lung Cell Mol. Physiol. 290, L32-40]
Phase 2 detoxifying (conjugating) enzymes are classified as "indirect" antioxidants based on their role in maintaining redox balance and thiol homeostasis. They contribute to biosynthesis, the recycling of thiols and facilitate the excretion of oxidized, reactive secondary metabolites (quinones, epoxides, aldehydes, and peroxides) through reduction/conjugation reactions during the process of xenobiotic detoxification. [Talalay, P., Holtzclaw, W. D., and Dinkova-Kostova, A. T. (2004) Importance of Phase 2 gene regulation in protection against electrophile and reactive oxygen toxicity and carcinogensis. Adv. Enzyme Regul. 44, 335-367]
Phase 2 enzymes with antioxidant capability include glutathione S-transferase isozymes and NADP(H):quinine oxidoreductase (NQO1), glutamyl cysteine ligase (GCLC), and UDP-glucuronosyltransferase (UGT).

The signal transduction pathways responsible for sensing oxidative stress and activating the appropriate defense genes are still not completely understood in mammals. The transcription factor, nuclear factor erythroid 2-related factor 2 (Nrf2), which is activated by ROS, appears to be a key regulator in oxidative stress gene regulation. Nrf2 is a member of the Cap'n'Collar family of bZIP proteins and recognizes the antioxidant response element (ARE) in the promoter of its over 2000 target genes. Under normal basal conditions, Nrf2 is bound to its inhibitor, the cytoskeleton-associated protein Keap1, which represses Nrf2 by facilitating its proteasomal degradation. [Itoh, K., Wakabayashi, N., Katoh, Y., Ishii, T., Igarashi, K., Engel, J. D., and Yamamoto, M. (1999) Keap1 represses nuclear activation of antioxidant responsive elements by Nrf2 through binding to the amino-terminal Neh2 domain. Genes Dev. 13, 76-86] Upon treatment by antioxidants, Nrf2 is released from Keap1 and translocates into the nucleus, followed by heterodimerization with other transcription factors, such as Jun and small Maf [Venugopal, R., and Jaiswal, A. K. (1998) Nrf2 and Nrf1 in association with Jun proteins regulate antioxidant response element-mediated expression and coordinated induction of genes encoding detoxifying enzymes. Oncogene 17, 3145-3156 ; Nguyen, T., Sherratt, P. J., and Pickett, C. B. (2003) Regulatory mechanisms controlling gene expression mediated by the antioxidant response element. Annu. Rev. Pharmacol. Toxicol. 43, 233-260] and initiates up-regulating of antioxidant genes.

*In vitro* gene expression has been used to understand the role of antioxidants in many diseases. Gene expression was used to show 200 differentially expressed oxidative genes in subjects with COPD (chronic obstructive pulmonary disorder) when compared with healthy smokers and the significant changes in oxidant response genes observed in vivo were reproduced in vitro using primary bronchial epithelial cells from the same donors. [Pierrou, S., Broberg, P., O'Donnell, R.A., Pawkowski, K., Virtala, R., Lindqvist, E. (2007) Expression of Genes Involved in Oxidative Stress Responses in Airway Epithelial Cells of Smokers with Chronic Obstructive Pulmonary Disease. Am. J. of Respiratory and Critical Care Medicine, 175(6), 577-587] In vitro gene expression has also been studied in human corneal endothelial cells (HCECs) to see if nuclear oxidative DNA damage increases with age and whether HCECs respond to this damage by upregulating their expression of oxidative stress and DNA damage-signaling genes in an agedependent manner. [Joyce, N.C., Harris, D.L., Zhu, C.C. Age-Related Gene Response of Human Corneal Endothelium to Oxidative Stress and DNA Damage (2011) Cornea, 52(3), 1641-1649] Four of 84 oxidation protective genes tested showed a statistically significant age-related difference in their expression.

Studies have also shown that diet can play a role in regulation of oxidative protective genes. A recent study aiming to identify molecular markers of diet-related diseases in response to food, evaluated peripheral blood mononuclear cells (PBMC) as a model in vitro system as a readily available source of RNA to discern gene expression signatures in relation to personalized obesity treatment. PBMC were collected from obese men before and after an 8-week low-calorie diet (LCD) to lose weight. Changes in gene expression before and after the LCD were validated by qRT-PCR and showed a decrease in some specific oxidative stress and inflammation genes. [Crujeiras, A., Parra, D., Milagro, F., Goyenechea, E., Larrarte, E., Margareto, J., Martinez, A. (2008) Differential Expression of Oxidative Stress and Inflammation Related Genes in Peripheral Blood Mononuclear Cells in Response to a Low-Calorie Diet: A Nutrigenomics Study. OMICS: A Journal of Integrative Biology, 12(4), 1-12]

No regulation of oxidation protective genes has been reported by orally ingested protein hydrolysates and fish protein hydrolysates, in particular. As the major site of first entry for xenobiotics, the oral cavity and the gastrointestinal tract are continuously exposed to a broad array of compounds with ROS capability. Further mucosal metabolism can lead to metabolites with increased toxicity, increasing the susceptibility of the oral cavity and gastrointestinal tract to oxidative metabolites, chemical toxicity, and potential necrotizing colitis. This oxidative damage can also be observed as a secondary effect in nerve tissue as part of the central nervous system.

The up regulation of oxidation protective genes is a viable biological target that would be able to confer a protective effect on the gastrointestinal tract and the central nervous system which can be used in conjunction with medical treatments in the control of Inflammatory Bowel Diseases (IBD) such as ulcerative colitis and Crohn's disease and CNS degenerative disease such as Parkinson's, multiple sclerosis and Alzheimer's diseases.

Accordingly, there still exists a need to develop an ingestible treatment for the oxidative protection of various organs and tissues, when taken orally. The present invention fulfills this need and provides further related advantages.

### Summary of the invention:

The present invention provides a fish protein hydrolysate powder and its constituent peptides for oral use in regulating the function of human oxidation protective genes which provide protection against oxidative damage in various organs and tissues. The fish protein hydrolysate powder according to the present invention compromise one or more peptide compounds with oxidation protective gene regulating activity incorporated into an oral formulation.

The fish protein hydrolysate powder according to the present invention possesses improved efficacy for protecting the gastro-intestinal and central nervous system from oxidative damage. Compositions comprising the fish protein hydrolysate powder according to the present invention may optionally contain antioxidants or free radical scavengers selected from vitamin E, vitamin A, tree tea oil, green tea extract, butylated hydroxyl anisole (BHA), butylated hydroxyl toluene (BHT) and ferulic acid or derivatives thereof, and the like.

Compositions comprising the fish protein hydrolysate powder according to the present invention can further comprise a food grade acceptable ingredient suitable for use in such preparations, such ingredient may include flavorings, emulsifiers, surfactants, emollients, essential oils, gelling agents, humectants, colorants and the like.

Furthermore, the composition comprising the fish protein hydrolysate powder according to the present invention can be in the form of a powder or solution that facilitates oral delivery for babies, infants, adults and older adults who may have difficulty in swallowing.

The present invention also includes a method for treating gastro-intestinal and central nervous system diseases potentially caused by oxidative damage to the organs and tissues concerned. The skin composition according to the present invention can be used to protect the gastro-intestinal and central nervous system from oxidative damage, by orally ingesting an effective amount of the fish protein hydrolysate or one or more of its constituent peptides.

### Detailed Description Of The Invention:

Before describing the present invention in detail, it is to be understood that the terminology used herein is for the purpose of describing particular embodiments only, and is not intended to be limiting. It must be noted that, as used herein and in the appended claims, the singular forms "a," "an," and "the" include plural referents unless the context clearly indicates otherwise.

The term "salmonids" as used herein means any fish of the family Salmonidae. The term "fish protein hydrolysate" as used herein means the resultant peptides formed from enzymatic hydrolysis of any fish organism or parts thereof. The term "enzymatic hydrolysis" used herein means the process of using protease enzymes, both natural and artificial, fixed or free, endo and exo, to hydrolyze proteins into constituent amino acids, peptides and oligopeptides. The terms "endo and exo" as used herein means protease enzymes that hydrolyze proteins from internal amino acid bonds and terminal amino acid bonds, respectively. The term protein hydrolysate and protein hydrolysate powder are hereinafter used interchangeably.

In accordance with the present invention, there is provided a fish protein hydrolysate powder and a composition comprising said powder, for oral use, to protect the gastro-intestinal and central nervous system from oxidative damage.

The fish protein hydrolysate comprises one or more bioactive peptide compounds, particularly one or more peptides capable of regulating the expression of one or more oxidation protective genes that confer protection to the gastro-intestinal and central nervous systems.

Use of protein hydrolysates in delivering protein which is easily digested and bioavailable have been reported. However, the use of such protein hydrolysates to specifically increase protection from oxidative damage has not been reported. More specifically, oral delivery of active peptides via a protein hydrolysate, into the gastro-intestinal tract that can regulate oxidation protective genes, such as those present in the Nrf2 pathway, have not been described in the literature.

Thus, one aspect of the present invention is directed to provide a fish protein hydrolysate which modifies the regulation of oxidation protective genes, such as those found in the Nrf2 cascade, to provide protection of the gastro-intestinal and central nervous systems, so that the subject who is being treated with the said hydrolysate gets the maximum benefit of enhanced efficacy.

The hydrolysate powder or composition according to the present invention comprises of a fish protein hydrolysate containing one or more peptides prepared as powder and delivered orally as a solution in water or any other liquid food such as juices, milk or aerated drinks.

Those skilled in the art know that a protein hydrolysate can be achieved by acidic, basic and enzymatic means and that the resultant protein hydrolysate will have different characteristics and constituents. Further, hydrolysis by enzymatic means will lead to different peptide constituents and these are dependent on the original protein source and the enzymes used. For the purpose of the present invention, the hydrolysis is limited to enzymatic hydrolysis, the protein source is limited to fish and the enzymes are limited to protease enzymes. The preferred protein source is fish of the salmonid family, particularly salmon (*salmo salar*) offcuts, heads, backbones, tail and skin that result after the filleting process. The preferred enzymes are endo and exo proteases sourced from microbial sources and with a DAPU (detergent alkaline protease units) number between 200 and 1000 ml/g or a natural protease activity measured between 100,000 and 500,000 PC/g.

It has surprisingly been found that the enzymatic hydrolysis process according to the invention provides a fish protein hydrolysate and a fish protein hydrolysate powder that are biologically active and may be used as a medicament.

Thus, one aspect of the present invention is directed to an enzymatic hydrolysis process for producing a fish protein hydrolysate powder, comprising the consecutive steps of:
i) mixing grinded fish protein material with water and heating;
ii) adding endopeptidase enzyme to the mixture of i) and stirring;
iii) adding exopeptidase enzyme in the form of carboxypeptidase to the mixture of ii) and stirring;
iv) stopping the enzymatic hydrolysis by heat inactivation;
v) the fish hydrolysate fraction and the remaining solid material is separated by filtration; and
vi) concentrating and drying the fish hydrolysate fraction s to yield a fish protein hydrolysate powder.

The fish protein material may originate any fish, peferably a fish of the salmonid family, particularly salmon (*salmo salar*)*.* The protein material may be any fish material such as muscle, offcuts, heads, backbones, tail and skin that result after the filleting process or any combinations thereof.

Grinding of the fish protein material may be performed by any well-known procedure. The ratio of grinded fish mass to water may be in the range from 1:0.5 to 1:5, preferably 1:1 by weight.

The endopeptidease (first enzyme) used may be any endopeptidase or combination thereof. Preferred endopetidases are pepsin and trypsin or any combination thereof.

The exopeptidase (second enzyme) used are selected from the group of carboxypeptidases, and any carboxypeptidase or combination thereof may be used. The amount of added endopeptidase and exopeptidase enzymes may be in the range from 0.05 to 1 % wt/wt of fish mass.

One embodiment of the process according to the invention further comprises a third hydrolysis step of adding a protease enzyme derived from aspergillus oryzae to the mixture of step iii) above. The third protease enzyme may be added in a ratio of 0.01 to 0.1% wt/wt fish mass.

The temperature of the enzymatic hydrolysis steps are adjusted to a convenient temperature at which the selected enzymes are active. Preferably the temperature range of the hydrolysis steps, i.e. steps i) - iv) are 35 °C - 60 °C, more preferably 50 °C. The duration of action of the first, second and third enzymes may be from 10 to 30 minutes for each. The protein hydrolysate obtained by subjecting the hydrolysate mixture to a sequencial addition of a first, second and third enzyme provides a palatable and biologically active hydrolysate. The protein hydrolysate obtained by subjecting the hydrolysate mixture to just a first and second enzyme provides a less palatable, but still biologically active hydrolysate.

In case the hydrolysis mixture is subjected to just a first and second enzyme, the time of action of one or both enzymes are prolonged. In one embodiment, the time of actions of the first, second and third enzyme are 30 min, 15 min and 10 min, respectively.

In another embodiment, the time of actions of the first and second enzyme are 30 min and 25 min, respectively.

The enzymatic hydrolysis is stopped by heat inactivation, i.e. the temperature of the hydrolysis mixture is increased to a temperature wherein the enzymes are no longer active. Preferably the hydrolysis mixture is heated to a temperature ranging from 80 °C to 95 °C, more preferably 85 °C. For the inactivation to be sufficient, the temperature must be maintained at the inactivation temperature for a sufficient time, such as e.g. 15 min.

The separation of the protein hydrolysate fraction and the remaining solid material may be performed by any suitable filtration or centrifugation technique. A suitable example is vibrating sieve with variable mesh sizes. Following filtration, the fish protein hydrolysate fraction is concentrated and dried to obtain the final fish protein hydrolysate powder. Any suitable concentration and drying processes may be used. As an example, the hydrolysate fraction is concentrated to 30 % dry matter in a conventional evaporator and spray-dried to yield the suitable final dry matter content. Preferably the fish protein hydrolysate powder has a final dry matter content of 95 - 99 %, more preferably 98%.

The enzymatic hydrolysis process may further include processing aids such as anti-foaming agents or surfactants or any combination thereof.

Another aspect of the present invention is a fish protein hydrolysate powder obtainable by the process according to the invention for use as a medicament.

A benefit of the fish protein hydrolysate powder provided according to the invention is that it is formed by enzymatic hydrolysis using non-GMO microbial protease enzymes.

Another aspect of the present invention is directed to a composition comprising the fish protein hydrolysate powder according to the invention together with at least one pharmacologically acceptable carrier or excipient for use as a medicament.

In one embodiment, the composition further comprises additional protein and protein hydrolysates of animal and plant sources or any combination thereof.

In another embodiment, the composition further comprises an antioxidant or a free radical scavenger selected from vitamin E, vitamin A, tree tea oil, green tea extract, butylated hydroxyl anisole (BHA), butylated hydroxyl toluene (BHT), or any combinations thereof.

In yet another embodiment, the composition further comprises carbohydrates, flavors and sweeteners.

In yet another embodiment, the composition further comprises suitable humectants or emulsifiers or any combination thereof.

It has surprisingly been found that the enzymatic hydrolysis process according to the invention provides a fish protein hydrolysate and a fish protein hydrolysate powder that are biologically active and may be used as a medicament.

Thus, one embodiment of the present invention is directed to a fish protein hydrolysate powder or a composition according to the invention, for use in the prophylaxis or treatment of gastrointestinal disorder or disease wherein the gastro-intestinal disorder or disease are selected from the group comprising gastro-esophageal reflux disease, gastro-enteritis, irritable bowel syndrome, enterocolitis, coeliac disease and proctitis diseases.

Another embodiment of the present invention is directed to a fish protein hydrolysate powder or a composition according to the invention, for use in the prophylaxis or treatment of nervous system disorders or diseases, wherein the central nervous system disorder or disease are selected from the group comprising neurodegenerative diseases, Alzheimer disease, Parkinson disease, amyotrophic lateral sclerosis, cerebrovascular disorders, demyelinating diseases, and psychiatric disorders.

Another embodiment of the present invention is directed to a fish protein hydrolysate powder or a composition according to the invention, for use in the prophylaxis or treatment of gastrointestinal or central nervous system disorders or diseases by modifying the expression of oxidation protective genes.

Another embodiment of the present invention is directed to a fish protein hydrolysate powder or a composition according to the invention, for use in the prophylaxis or treatment of gastrointestinal or central nervous system disorders or diseases by modifying the expression of oxidation protective genes, wherein the oxidation protective genes are present within the nuclear factor erythroid 2-related factor 2 (Nrf2) and regulated set of genes.

Another embodiment of the present invention is directed to a fish protein hydrolysate powder or a composition according to the invention, for use in the prophylaxis or treatment of gastrointestinal or central nervous system disorders or diseases by modifying the expression of oxidation protective genes, wherein the oxidation protective genes are Apolipoprotein E, Cytoglobin, Eosinophil peroxidase, Ferritin, heavy polypeptide 1, Glutamine-cysteine ligase, Glutathione peroxidase 1, Glutathione synthetase, Glutathione transferase zeta 1, Heme oxygenase 1, Mannose binding lectin 2, Methionine sulfoxide reductase A, Nitric oxide synthase 2, NAD(P)H dehydrogenase quinone 1, Peroxiredoxin 5, Selenoprotein S, Superoxide dismutase 1, Arachidonate 12-lipoxygenase, Epoxide hydrolase 2, Microsomal glutathione S-transferase 3, Myeloperoxidase, Neutrophil cytosolic factor 1, NADPH oxidase, Peroxiredoxin 1, Prostaglandin endoperoxide synthase 2, Sulfiredoxin

Other features of the invention will become apparent in the course of the following descriptions of exemplary embodiments, which are given for illustration of the invention and are not intended to be limiting thereof.

### Example 1 - Preparation of biologically active fish hydrolysate powder according to the invention

Salmon Protein Hydrolysate powder is produced by enzymatic hydrolysis of salmon (salmo salar) head and backbone post filleting. 1000 grams of ground head and backbone is added to 1000 ml of water and the mixture heated to 50 °C. To this is added 10 g of an endopeptidase enzyme - pepsin - and stirred for 30 minutes. To this is further added 10 g of an exopeptidase enzyme - carboxypeptidase - and stirred for 15 minutes. To this is further added 5 grams of enzyme Flavourzyme(R) (an protease derived from aspergillus oryzae) and the mixture stirred for 10 minutes. The entire mixture is then heated to 85 °C, maintained at said temperature for 15 minutes and filtered. The hydrolysate fraction is concentrated to 30 % dry matter in a conventional evaporator and spray-dried to yield the fish protein hydrolysate powder according to the invention as a light yellow free flowing powder with the biological efficacy shown in examples 6-11 herein.

### Example 2 - Preparation of fish hydrolysate powder with no biological effect

Salmon Protein Hydrolysate powder is produced as set forth in Ex 1 above, except that the exopeptidase used is an aminopeptidase instead of a carboxypeptidase. A powder with no biological efficacy was obtained (data not shown).

In detail; Salmon Protein Hydrolysate powder is produced by enzymatic hydrolysis of salmon (salmo salar) head and backbone post filleting. 1000 grams of ground head and backbone is added to 1000 ml of water and the mixture heated to 50 °C. To this is added 10 g of an endopeptidase enzyme - pepsin - and stirred for 30 minutes. To this is further added 10 g of an exopeptidase enzyme - aminopeptidase - and stirred for 15 minutes. To this is further added 5 grams of enzyme Flavourzyme(R) and the mixture stirred for 10 minutes. The entire mixture is then heated to 85 °C, maintained at said temperature for 15 minutes and filtered. The aqueous layer is concentrated to 30 percent dry matter in a conventional evaporator and spray-dried to yield the a powder with no biological efficacy.

### Example 3 - Preparation of fish hydrolysate powder with no biological effect

Salmon Protein Hydrolysate powder is produced as set forth in Ex. 1 above, except that the exopeptidase (carboxypeptidase) is added prior to endopeptidase. A powder with no biological efficacy was obtained (data not shown).

In detail; Salmon Protein Hydrolysate powder is produced by enzymatic hydrolysis of salmon (salmo salar) head and backbone post filleting. 1000 grams of ground head and backbone is added to 1000 ml of water and the mixture heated to 50 °C. To this is added 10 g of an exopeptidase enzyme - carboxypeptidase - and stirred for 30 minutes. To this is further added 10 g of an endopeptidase enzyme - pepsin - and stirred for 15 minutes. To this is further added 5 grams of enzyme Flavourzyme(R) and the mixture stirred for 10 minutes. The entire mixture is then heated to 85 °C, maintained at said temperature for 15 minutes and filtered. The aqueous layer is concentrated to 30 percent dry matter in a conventional evaporator and spray-dried to yield the a powder with no biological efficacy.

### Example 4 - Preparation of biologically active fish hydrolysate powder according to the invention

Salmon Protein Hydrolysate powder is produced as set forth in Ex. 1 above, except that the addition of the third enzyme is omitted and the time of action of the exopeptidase is prolonged. A powder with the biological efficacy shown in examples 6-11 herein was obtained.

In detail; Salmon Protein Hydrolysate powder is produced by enzymatic hydrolysis of salmon (salmo salar) head and backbone post filleting. 1000 grams of ground head and backbone is added to 1000 ml of water and the mixture heated to 50 °C. To this is added 10 g of an endopeptidase enzyme - pepsin - and stirred for 30 minutes. To this is further added 10 g of an exopeptidase enzyme - carboxypeptidase - and stirred for 25 minutes. The entire mixture is then heated to 85 °C, maintained at said temperature for 15 minutes and filtered. The aqueous layer is concentrated to 30 percent dry matter in a conventional evaporator and spray-dried to yield the fish protein hydrolysate powder according to the invention as a light yellow free flowing powder with the biological efficacy shown in examples 6-11 herein.

### Example 5 - Preparation of biologically active fish hydrolysate powder according to the invention

Salmon Protein Hydrolysate powder is produced as set forth in Ex 1 above, except that the endopeptidase enzymes used is trypsin instead of pepsin. A powder with the biological efficacy shown in examples 6-11 herein was obtained.

In detail; Salmon Protein Hydrolysate powder is produced by enzymatic hydrolysis of salmon (salmo salar) head and backbone post filleting. 1000 grams of ground head and backbone is added to 1000 ml of water and the mixture heated to 50 °C. To this is added 10 g of an endopeptidase enzyme - trypsin - and stirred for 30 minutes. To this is further added 10 g of an exopeptidase enzyme - carboxypeptidase - and stirred for 15 minutes. To this is further added 5 grams of enzyme Flavourzyme(R) and the mixture stirred for 10 minutes. The entire mixture is then heated to 85 °C, maintained at said tempearture for 15 minutes and filtered. The aqueous layer is concentrated to 30 percent dry matter in a conventional evaporator and spray-dried to yield the desired powder with the biological efficacy shown in examples 6-11 herein.

By the above examples it has surprisingly been shown, that the sequence of adding the hydrolytic enzymes as well as the nature of the enzymes are of importance to obtain a biologically efficient hydrolysate powder. The fish protein material is first subjected to an endopeptidase, thereafter subjected to an exopeptidase in form of a carboxypeptidase. The nature of the endopeptidase has been shown to be of less importance. Optionally, the hydrolysate may be subjected to a third protease derived from aspergillus oryzae to obtain a hydrolysate with improved palatability.

In the following Examples 6 - 11, the biological activity of Salmon Protein Hydrolysate Powder obtained by the process according to the invention was tested, hereinafter identified SPH.

### Example 6

Salmon Protein Hydrolysate Powder (SPH) according to the invention was tested in the following experiment. HGEPp (primary pooled human gingival epithelial cells) cells were purchased from CellnTec Advanced Cell Systems AG. The RNEasy Plus Micro Kit, RNase-free DNase Set, RT² Easy First Strand Kit (DNA generator) and RT² SYBR^{®} Green fluor qPCR mastermix were purchased from Qiagen N.V., USA. Oxidative Stress RT² profiler PCR arrays (84 protective genes) were purchased from Qiagen N.V., USA. The iCycler PCR system from Bio-Rad Inc., USA was used for the RT-PCR.

Pooled primary HGEPp cells were propagated in CnT-Prime epithelial culture medium provided by CellnTec on 100 mm petri dishes coated with 30 mg/ml Type I rat tail collagen (BD Biosciences) diluted in Dulbecco's phosphate-buffered saline (DPBS). Cell density of 2.5×10⁴ cells/cm² was used to grow cell monolayers and acclimated overnight at 37°C.

The HGEPp cells were grown in 4 × 60 mm Nunc^{™} Cell Culture dishes. at 2.5×10⁴ cells/cm² density concentration. SPH 100 mM DMSO stock suspension was prepared. Further dilutions were prepared of 25, 50, and 100 µM/ml of SPH. All dosing solutions contained 0.3% of DMSO which is below the maximum tolerated DMSO concentration of 0.8% for HGEPp cells.

Dish cell concentrations were selected to be 2.5×10⁵ /ml to yield an OD absorbance within the linear portion of the control curve for both cells lines. Six HGEPp and cell culture dishes were pretreated for 24 h with SPH at 25, 50 and 100 g/mL concentrations (in duplicate) at 37°C. One cell culture dish for each cell line was pretreated for 24 hrs with the DMSO blank solution at 37°C.

The RNeasy UCP Micro Kit was used to purify mRNA from both HGEPp treated cells. After discarding and washing treatments, the cells were pelleted by centrifugation for 5 min at 1000 RPM in a centrifuge tube. All the supernatant was carefully removed by aspiration, making sure all the cell medium has been removed thoroughly. The cells were disrupted by adding 350 µl buffer RULT taking care to loosen the cell pellet from the tube and vortexed to mix thoroughly and the mixture was homogenized by passing the lysate 5 times through 20-gauge needle fitted to an RNase-free syringe. Added 350 µl of 70% ethanol to the lysate, and mixed again by pipetting. Transferred the sample, including any precipitate that may have formed, to an RNeasy UCP MinElute spin column placed in a 2 ml collection tube and centrifuged for 15 s at 10,000 rpm. Finally eluted with 16 µl ultra-clean water to yield a 20 µl (4 µg) RNA eluate.

A RNA sample from each of the above seven treatments was added to 40 µl of Buffer GE2 (gDNA elimination buffer) and RNase-free H2O to make a final volume of 60 µl. Incubated at 37°C for 5 min and immediately placed on ice for 2 minutes. Added 62 µl of the BC5 Reverse Transcriptase Mix to each 60 µl RNA sample for a final volume of 102 µl. Incubated at 42°C for exactly 15 minutes and then immediately stopped the reaction by heating at 95°C for 5 minutes. Sample is ready for PCR.

RT-PCR was used to analyze expression levels of 84 genes related to oxidative stress in HIEC-6 cells pre-treated with SPH at 25, 50 and 100 g/ml for 24 has shown in Example 3. Gene expression was compared using Ct values and the results were calculated using ΔΔ Ct method with normalization to the average expression levels of the five common genes (ACTB, B2M, GAPDH, HPRT, and RPL13A).

The selection of the 96 well array of test genes and housekeeping genes are shown in below.

### ANTIOXIDANTS

Glutathione Peroxidases (GPx):
   GPX1, GPX2, GPX3, GPX4, GPX5, GPX6, GPX7, GSTP1, GSTZ1.
Peroxiredoxins (TPx):
   PRDX1, PRDX2, PRDX3, PRDX4, PRDX5, PRDX6.
Other Peroxidases:
   CAT, CYBB, CYGB, DUOX1, DUOX2, EPX, LPO, MGST3, MPO, PTGS1 (COX1), PTGS2 (C OX2), PXDN, TPO, TTN.
Other Antioxidants:
   ALB, APOE, GSR, MT3, SOD1, SOD3, SRXN1, TXNRD1, TXNRD2, VIMP.

### REACTIVE OXYGEN SPECIES (ROS) METABOLISM

Superoxide Dismutases (SOD):
   SOD1, SOD2, SOD3.
Other Superoxide Metabolism Genes:
   ALOX12, CCS, DUOX1, DUOX2, GTF2I, MT3, NCF1, NCF2, NOS2, NOX4, NOX5, PREX1, UCP2.
Other Reactive Oxygen Species (ROS) Metabolism Genes:
   AOX1, BNIP3, EPHX2, MPV17, SFTPD.
Oxidative Stress Responsive Genes:
   APOE, ATOX1, CAT, CCL5, CYGB, DHCR24, DUOX1, DUOX2, DUSP1, EPX, FOXM1, FT H1, GCLC, GCLM, GPX1, GPX2, GPX3, GPX4, GPX5, GPX6, GPX7, GSR, GSS, HMOX1, H SPA1A, KRT1, LPO, MBL2, MPO, MSRA, NQO1, NUDT1, OXR1, OXSR1, PDLIM1, PNKP, PRDX2, PRDX5, PRDX6, PRNP, RNF7, SCARA3, SEPP1, SIRT2, SOD1, SOD2, SQSTM1, S RXN1, STK25, TPO, TTN, TXN, TXNRD1, TXNRD2, VIMP

### OXYGEN TRANSPORTERS

CYGB, MB

### Example 7

Salmon Protein Hydrolysate Powder (SPH) according to the invention was tested in the following experiment. HIEC-6 (human intestinal epithelial cells) cells were purchased from the ATCC, USA. The RNEasy Plus Micro Kit, RNase-free DNase Set, RT² Easy First Strand Kit (DNA generator) and RT² SYBR^{®} Green fluor qPCR mastermix were purchased from Qiagen N.V., USA. Oxidative Stress RT² profiler PCR arrays (84 protective genes) were purchased from Qiagen N.V., USA. The iCycler PCR system from Bio-Rad Inc., USA was used for the RT-PCR.

HIEC-6 (ATCC CRL-3266) cells were propagated in OptiMEM 1 reduced serum medium (Gibco Catalog No. 31985) with 20 mM HEPES, 10 mM GlutaMAX, 10 ng/ml Epidermal Growth Factor (EGF) and fetal bovine serum to a final concentration of 4%, on 100 mm petri dishes. Cell density of 1×10⁵ cells/cm² was used to grow cell monolayers and acclimated overnight at 37°C.

The HIEC-6 cells were grownin 4 × 60 mm Nunc^{™} Cell Culture dish. SPH 100 mM DMSO stock suspension was prepared. Further dilutions of 25, 50, and 100 µM/ml of SPH were prepared. All dosing solutions contained 0.3% of DMSO which is below the maximum tolerated DMSO concentration of 0.8% for HIEC-6 cells.

Dish cell concentrations were selected to be 2.5×10⁵ /ml to yield an OD absorbance within the linear portion of the control curve for both cells lines. Six HIEC-6 cell culture dishes were then pretreated for 24 h with SPH at 25, 50 and 100 g/mL concentrations (in duplicate) at 37°C. One cell culture dish for each cell line was pretreated for 24 hrs with the DMSO blank solution at 37°C.

The RNeasy UCP Micro Kit was used to purify mRNA from both HGEPp treated cells. After discarding and washing treatments, the cells were pelleted by centrifugation for 5 min at 1000 RPM in a centrifuge tube. All the supernatant was carefully removed by aspiration, making sure all the cell medium has been removed thoroughly. The cells were disrupted by adding 350 µl buffer RULT taking care to loosen the cell pellet from the tube and vortexed to mix thoroughly and the mixture was homogenized by passing the lysate 5 times through 20-gauge needle fitted to an RNase-free syringe. Added 350 µl of 70% ethanol to the lysate, and mixed again by pipetting. Transferred the sample, including any precipitate that may have formed, to an RNeasy UCP MinElute spin column placed in a 2 ml collection tube and centrifuged for 15 s at 10,000 rpm. Finally eluted with 16 µl ultra-clean water to yield a 20 µl (4 µg) RNA eluate.

A RNA sample from each of the above seven treatments was added to 40 µl of Buffer GE2 (gDNA elimination buffer) and RNase-free H2O to make a final volume of 60 µl. Incubated at 37°C for 5 min and immediately placed on ice for 2 minutes. Added 62 µl of the BC5 Reverse Transcriptase Mix to each 60 µl RNA sample for a final volume of 102 µl. Incubated at 42°C for exactly 15 minutes and then immediately stopped the reaction by heating at 95°C for 5 minutes. Sample is ready for PCR.

RT-PCR was used to analyze expression levels of 84 genes related to oxidative stress in HIEC-6 cells pre-treated with SPH at 25, 50 and 100 g/ml for 24 has shown in Example 3. Gene expression was compared using Ct values and the results were calculated using ΔΔ Ct method with normalization to the average expression levels of the five common genes (ACTB, B2M, GAPDH, HPRT, and RPL13A).

### Example 8

### Upregulated oxidative stress genes

Treatment of HGEPp cells with SPH showed tip-regulation with a Fold Change greater than two for sixteen human oxidative stress-related genes - APOE, CYGB, EPX, FTH1, GCLC, GPX1, GSR, GSTZ1, HMOX1, MBL2, MSRA, NOS2, NQO1, PRDX5, SELS, SOD1 while treatment of HIEC-6 cells with SPH showed up-regulation with a Fold Change greater than two for eleven human oxidative stress-related genes - APOE, EPX, FTH1, GCLC, GSS, HMOX1, MBL2, NOS2, NQO1, PRDX5, SOD1 at the 100 µM/ml SPH concentration (Table 1 & 2). The DMSO blank treatments did not show any change in regulation for any gene in the array. Ten genes showed common upregulation in both cell lines - APOE, EPX, FTH1, GCLC, HMOX1, MBL2, NOS2, NQO1, PRDX5, SOD1.

**Table 1**

| Upregulated oxidative stress-related genes in HGEPp cells following SPH treatment | | | | |
|---|---|---|---|---|
| GeneBank | Symbol | Description | Fold Change | P value |
| NM_000041 | APOE | Apolipoprotein E | 2.76 | 0.028 |
| NM_134268 | CYGB | Cytoglobin | 2.59 | 0.041 |
| NM_000502 | EPX | Eosinophil peroxidase | 3.05 | 0.037 |
| NM_002032 | FTH1 | Ferritin, heavy polypeptide 1 | 4.82 | 0.033 |
| NM_001498 | GCLC | Glutamine-cysteine ligase cat. sub. | 4.91 | 0.016 |
| NM_000581 | GPX1 | Glutathione peroxidase 1 | 2.88 | 0.034 |
| NM_000178 | GSS | Glutathione synthetase | 2.63 | 0.011 |
| NM_001513 | GSTZ1 | Glutathione transferase zeta 1 | 2.49 | 0.045 |
| NM_002133 | HMOX1 | Heme oxygenase (decycling) 1 | 5.63 | 0.017 |
| NM_000250 | MBL2 | Mannose binding lectin 2 | 2.42 | 0.028 |
| NM_012331 | MSRA | Methionine sulfoxide reductase A | 2.07 | 0.034 |
| NM_000625 | NOS2 | Nitric oxide synthase 2 | 2.76 | 0.026 |
| NM_000903 | NQO1 | NAD(P)H dehydrogenase quinone 1 | 3.85 | 0.019 |
| NM_181652 | PRDX5 | Peroxiredoxin 5 | 2.74 | 0.039 |
| NM_203472 | SELS | Selenoprotein S | 2.26 | 0.042 |
| NM_000454 | SOD1 | Superoxide dismutase 1 | 3.50 | 0.013 |

**Table 2**

| Up regulated oxidative stress-related genes in HIEC-6 cells following SPH treatment | | | | |
|---|---|---|---|---|
| GenBank | Symbol | Description | Fold Change | P Value |
| NM_000041 | APOE | Apolipoprotein E | 5.43 | 0.037 |
| NM_000502 | EPX | Eosinophil peroxidase | 2.79 | 0.022 |
| NM_002032 | FTH1 | Ferritin, heavy polypeptide 1 | 4.25 | 0.039 |
| NM_001498 | GCLC | Glutamine-cysteine ligase cat. sub. | 3.16 | 0.034 |
| NM_000178 | GSS | Glutathione synthetase | 2.99 | 0.020 |
| NM_002133 | HMOX1 | Heme oxygenase (decycling) 1 | 6.24 | 0.012 |
| NM_000250 | MBL2 | Mannose binding lectin 2 | 3.57 | 0.033 |
| NM_000625 | NOS2 | Nitric oxide synthase 2 | 2.48 | 0.041 |
| NM_000903 | NQO1 | NAD(P)H dehydrogenase quinone 1 | 3.30 | 0.029 |
| NM_181652 | PRDX5 | Peroxiredoxin 5 | 2.52 | 0.025 |
| NM_000454 | SOD1 | Superoxide dismutase 1 | 3.99 | 0.038 |

### Downregulated oxidative stress genes

Treatment of HGEPp cells with SPH showed downregulation with a Fold Change less than 50% for nine human oxidative stress-related genes - ALOX12, EPHX2, MGST3, MPO, NCF1, NOX5, PRDX1, PTGS2, SRXN1 while treatment of HIEC-6 cells with SPH showed downregulation with a Fold Change less than 50% for seven human oxidative stress-related genes (ALOX 12, MGST3, MPO, NCF1, NOX5, PTGS2, SRXN1) at the 100 µM/ml SPH concentration (Tables 3 & 4). Seven common genes showed downregulation in both cell lines - ALOX12, MGST3, MPO, NCF1, NOX5, PTGS2, SRXN1.

**Table 3**

| Downregulated oxidative stress-related genes in HGEPp cells following SPH treatment | | | | |
|---|---|---|---|---|
| GenBank | Symbol | Description | Fold Change | P Value |
| NM_000697 | ALOX12 | Arachidonate 12-lipoxygenase | 0.29 | 0.040 |
| NM_001979 | EPHX2 | Epoxide hydrolase 2 | 0.38 | 0.022 |
| NM_004528 | MGST3 | Microsomal glutathione S-transferase 3 | 0.46 | 0.031 |
| NM_000250 | MPO | Myeloperoxidase | 0.37 | 0.046 |
| NM_000265 | NCF1 | Neutrophil cytosolic factor 1 | 0.43 | 0.015 |
| NM_016931 | NOX5 | NADPH oxidase, Ca binding domain 5 | 0.41 | 0.023 |
| NM_002574 | PRDX1 | Peroxiredoxin 1 | 0.40 | 0.034 |
| NM_000963 | PTGS2 | Prostaglandin endoperoxide synthase 2 | 0.35 | 0.027 |
| NM_080725 | SRXN1 | Sulfiredoxin 1 | 0.42 | 0.038 |

**Table 4**

| Downregulated oxidative stress-related genes in HIEC-6 cells following SPH treatment | | | | |
|---|---|---|---|---|
| GenBank | Symbol | Description | Fold Change | P Value |
| NM_000697 | ALOX12 | Arachidonate 12-lipoxygenase | 0.17 | 0.023 |
| NM_004528 | MGST3 | Microsomal glutathione S-transferase 3 | 0.38 | 0.044 |
| NM_000250 | MPO | Myeloperoxidase | 0.29 | 0.035 |
| NM_000265 | NCF1 | Neutrophil cytosolic factor 1 | 0.40 | 0.019 |
| NM_016931 | NOX5 | NADPH oxidase, Ca binding domain 5 | 0.35 | 0.028 |
| NM_000963 | PTGS2 | Prostaglandin endoperoxide synthase 2 | 0.41 | 0.016 |
| NM_080725 | SRXN1 | Sulfiredoxin 1 | 0.34 | 0.022 |

### Dose dependent response genes

Additionally two upregulated genes, FTH1 and HMOX1 in HGEPp cells and three upregulated genes, APOE, FTH1 and HMOX1 in HIEC-6 cells showed a dose-dependent result at the two lower doses of 25 and 50 µM/ml of SPH treatment. The ALOX12 gene showed a dose dependent downregulation in both HGEP and HIEC-6 cells (Table 5). None of the other genes showed a greater than two fold change in upregulation nor a less than 50% change in down-regulation, when the SPH concentration was reduced to 50 and 25 µM/ml.

**Table 5**

| SPH dose-dependent gene regulation in both HGEP and HIEC-6 cells | | | | | |
|---|---|---|---|---|---|
| Fold Change at SPH concentrations of | | | | | |
| Genebank | Gene | Description | 25 µM/ml | 50 µM/ml | 100 µM/ml |
| HGEP Cells | | | | | |
| NM_002032 | FTH1 | Ferritin, heavy polypeptide | 2.10 | 2.96 | 4.82 |
| P value | | | 0.036 | 0.045 | 0.033 |
| NM_002133 | HMOX1 | Heme oxygenase (decycling) 1 | 1.92 | 3.47 | 5.63 |
| P value | | | 0.040 | 0.021 | 0.017 |
| NM_000697 | ALOX12 | Arachidonate 12-lipoxygenase | 0.49 | 0.37 | 0.29 |
| P value | | | 0.065 | 0.062 | 0.040 |

| HIEC-6 Cells | | | | | |
|---|---|---|---|---|---|
| NM_000041 | APOE | Apolipoprotein E | 2.20 | 3.12 | 5.43 |
| P value | | | 0.027 | 0.018 | 0.037 |
| NM_002032 | FTH1 | Ferritin, heavy polypeptide | 1.98 | 2.83 | 4.25 |
| P value | | | 0.039 | 0.032 | 0.039 |
| NM_002133 | HMOX1 | Heme oxygenase (decycling) 1 | 2.85 | 3.93 | 6.24 |
| P value | | | 0.027 | 0.018 | 0.012 |
| NM_000697 | ALOX12 | Arachidonate 12-lipoxygenase | 0.41 | 0.28 | 0.17 |
| P value | | | 0.047 | 0.034 | 0.023 |

### Example 9

Iron is required for normal cell growth and proliferation. However, excess iron is potentially harmful, as it can catalyze the formation of toxic reactive oxygen species (ROS). Thus cells have evolved highly regulated mechanisms for controlling intracellular iron levels. Chief among these is the sequestration of iron. The FTH1 gene encodes the heavy subunit of ferritin, the major intracellular iron storage protein in humans, which sequesters free iron in a soluble and nontoxic state from dietary sources of iron. Previous studies have shown that increased synthesis of both subunits of ferritin occurs when exposed to oxidative stress. Ferritin H or L overexpression reduced the accumulation of ROS in response to an oxidant challenge.

Example 3 shows that up regulation of FTH1 need not be solely in response to an oxidant challenge and may be induced by specific activator peptides that are present in salmon protein hydrolysate. FTH1 increase has been shown to result in a protective effect for neurological disorders such as Parkinson's Disease. (Rhodes, S.L., Ritz, B. (2008) Genetics of iron regulation and the role of iron in Parkinson's Disease Neurobiology of Disease, 32(2), 183-195)

### Example 10

HMOX1 gene expression is induced by oxidative stress and seems to confer cytoprotection. The resultant HO1 enzyme catalyzes the degradation of heme, which produces biliverdin, ferrous iron, and carbon monoxide. It cleaves the heme ring at the alpha-methene bridge to form biliverdin, which is converted to bilirubin by biliverdin reductase. Carbon monoxide released from heme oxygenase reactions has also been shown to influence vascular tone and the function of nitric oxide synthase.

The upregulation of HMOX1 as seen in the present invention in Example 3 shows that salmon protein hydrolysate when given enteraly has the ability, to reduce damage in the GI tract and be used for treatment of the neonatal intestinal inflammatory disease, necrotizing enterocolitis (NEC). HO-1 deficiency has been shown to lead to increased NEC development, while HO-1 induction increased Treg/Teff ratios and prevented NEC development in animal models [Schulz, S., Chisholm, K.M.,, Zhao, H., Kalish, F., Yang, Y., Wong, R.J., Stevenson, D.K. (2015) Heme oxygenase-1 confers protection and alters T-cell populations in a mouse model of neonatal intestinal inflammation. Pediatr Res. 77(5), 640-648].

Studies have also indicated that the ability to upregulate HO-1 expression as shown by salmon protein hydrolysate in Example 3, is an important protective factor in many diseases other as cardiovascular disease, renal transplantation, necrotizing enterocolitis [Otterbein, L.E., Soares, M.P., Yamashita, K , Bach, F.H. (2003) Heme oxygenase-1: unleashing the protective properties of heme. Trends in Immunology 24(8), 449-455]

### Example 11

Metabolic syndrome is a term used when the clustering of at least three of five of the following medical conditions occurs: abdominal (central) obesity, elevated blood pressure, elevated fasting plasma glucose (or overt diabetes), high serum triglycerides, and low high-density lipoprotein (HDL) levels.

ALOX12, a lipoxygenase-type enzyme, is encoded by the ALOX12 gene and is characterized by its ability to metabolize AA into 15(S)-HETE, a hormone-like autocrine and paracrine signaling agent, involved in inflammation response and metabolic syndrome. Elevated ALOX12 levels have been implicated in type 1 diabetes, in the fat cells of white adipose tissue of obese diabetic patients and in excessive production of reactive oxygen species and inflammation [Kuhn, H., Banthiya, S., van Leyen, K. (2015) Mammalian lipoxygenases and their biological relevance. Biochimica et Biophysica Acta (BBA) - Molecular and Cell Biology of Lipids. 1851(4), 308-330]. Down-regulation of ALOX12 and its metabolite(s) by SPH as seen in Example 3 can be used to contribute to the retardation of obesity, diabetes, hypertension, and/or metabolic syndrome.

Accordingly, it can be seen that a fish protein hydrolysate powder according to the present invention comprising amino acids, peptides, oligopeptides exhibits significant up regulation of several oxidation protective genes, particularly the FTH1 and HMOX1 gene and down regulation of several pro-inflammatory genes, particularly the ALOX12 gene, whose combined effects are known to confer protection from oxidative damage and when the fish protein hydrolysate of the said invention is delivered as an oral formulation, it will provide protection from gastro intestinal and central nervous system disorders such as irritable bowel syndrome, colitis, Parkinson's disease and Alzheimer's.

## Claims

1. Fish protein hydrolysate powder obtainable by an enzymatic hydrolysis process, comprising the consecutive steps of:
i) mixing grinded by-products from fish protein material with water and heating;
ii) adding endopeptidase enzyme to the mixture of i) and stirring;
iii) adding exopeptidase enzyme in the form of carboxypeptidase to the mixture of ii) and stirring;
iv) stopping the enzymatic hydrolysis by heat inactivation;
v) the fish hydrolysate fraction and the remaining solid material is separated by filtration; and
vi) concentrating and drying the fish hydrolysate fraction to yield a fish protein hydrolysate powder.
for use as a medicament

2. Fish protein hydrolysate powder according to claim 1, wherein said process further comprises a third hydrolysis step of adding protease enzyme derived from aspergillus oryzae to the mixture of iii) and stirring.

3. Fish protein hydrolysate powder according to claim 1, wherein the fish protein material originate from fish, preferably a salmonoids, more preferably *salmo salar.*

4. Fish protein hydrolysate powder according to claim 1, wherein the endopeptidase is selected from the group consisting of pepsin or trypsin, or any combination thereof.

5. Composition comprising the fish protein hydrolysate powder according to any of claims 1-4, together with at least one pharmacologically acceptable carrier or excipient for use as a medicament.

6. Fish protein hydrolysate powder according to claims 1-4 or composition according to claim 5, for use in the prophylaxis or treatment of gastrointestinal or central nervous system disorders or diseases.

7. Fish protein hydrolysate powder or composition according to claim 6, for use in the prophylaxis or treatment of gastrointestinal disorders or diseases wherein the gastrointestinal disorder or disease are selected from the group comprising gastro-esophageal reflux disease, gastro-enteritis, irritable bowel syndrome, enterocolitis, coeliac disease and proctitis diseases.

8. Fish protein hydrolysate powder or composition according to claim 6, for use in the prophylaxis or treatment of nervous system disorders or diseases, wherein the central nervous system disorder or disease are selected from the group comprising neurodegenerative diseases, Alzheimer disease, Parkinson disease, amyotrophic lateral sclerosis, cerebrovascular disorders, demyelinating diseases, and psychiatric disorders.
